# EUROPÄISCHE PATENTSCHRIFT

(11) **EP 0 885 183 B1**
(45) Veröffentlichungstag und Bekanntmachung des Hinweises auf die Patenterteilung: **20.06.2001**
(21) Anmeldenummer: 97902245.6
(22) Anmeldetag: 28.01.1997
(51) Int. Cl.: C07C 45/50, C07C 45/80, C07C 29/16, B01J 31/40, C07D 213/38, C07D 213/74

(54) **VERFAHREN ZUR HERSTELLUNG VON ALDEHYDEN DURCH HYDROFORMYLIERUNG VON OLEFINEN**
PROCESS FOR PRODUCING ALDEHYDES BY HYDROFORMYLATION OF OLEFINS
PROCEDE DE PREPARATION D'ALDEHYDES PAR HYDROFORMYLATION D'OLEFINES

(30) Priorität: 30.01.1996 DE 19603201
(43) Veröffentlichungstag der Anmeldung: 23.12.1998
(73) Patentinhaber: BASF AKTIENGESELLSCHAFT, 67056 Ludwigshafen (DE)
(72) Erfinder: PACIELLO, Rocco, D-67098 Bad Dürkheim (DE); KNEUPER, Heinz-Josef, D-68163 Mannheim (DE); GEISSLER, Bernhard, D-67281 Kirchheim (DE); RÖPER, Michael, D-67157 Wachenheim (DE)
(86) Internationale Anmeldenummer: EP9700372
(87) Internationale Veröffentlichungsnummer: WO9728113

(56) Entgegenhaltungen:
- US-A- 3 594 425
- US-A- 3 857 895
- US-A- 4 045 492
- CHEMIE INGENIEUR. TECHNIK., Bd. 44, Nr. 11, 1972, WEINHEIM DE, Seiten 708-712, XP002029846 B. FELL ET AL.: "Hydroformylierung mit Rhodiumcarbonyl-tert.-Amin-Komplexkatalysa toren"
- JOURNAL OF THE CHEMICAL SOCIETY, CHEMICAL COMMUNICATIONS, Bd. 18, 1992, LETCHWORTH GB, Seiten 1300-1302, XP002029847 R. GRIGG ET AL.: "Luminescent pH sensors based on di(2,2'-bipyridyl)(5,5'-diaminomethyl-2,2' -bipyridyl)-ruthenium(II) complexes"
- JOURNAL OF HETEROCYCLIC CHEMISTRY, Bd. 14, 1977, PROVO US, Seiten 191-194, XP002029848 C.P. WHITTLE: "A convenient synthetic route to 5,5'-disubstituted 2,2'-bipyridines"
- JOURNAL OF THE AMERICAN CHEMICAL SOCIETY, Bd. 80, 1958, DC US, Seiten 2745-2748, XP002029849 G. MAERKER ET AL.: "The synthesis of some 4,4'-disubstituted 2,2'-bipyridines"
- RECUEIL DES TRAVAUX CHIMIQUES DES PAYS-BAS, Bd. 112, 1993, AMSTERDAM NL, Seiten 351-357, XP002029850 J.G.J. WEIJNEN ET AL.: "Catalytic hydrolysis of phosphate esters by metallocomplexes of 1,10-phenanthroline derivatives in micellar solution"

## Beschreibung

Die Erfindung betrifft ein Verfahren zur Herstellung von Aldehyden oder Aldehyden und Alkoholen durch Hydroformylierung von Olefinen mit mehr als 3 Kohlenstoffatomen und Rückgewinnung des Katalysators durch eine Kombination von Rückführung des Destillationssumpfes nach Abdestillieren der Hydroformylierungsprodukte und Extraktion des Katalysatorkomplexes mit wäßrigen Lösungen schwacher Säuren, dessen mehrzähniger stickstoffhaltiger Ligand zusätzlich mindestens einen, mit schwachen Säuren protonierbaren tertiären Stickstoffrest enthält.

Die Hydroformylierung von Olefinen mit Kohlenmonoxid und Wasserstoff in Gegenwart von Übergangsmetall-Katalysatoren ist wohlbekannt. Während α-Olefine sehr gut mit rhodiumhaltigen phosphinmodifizierten Katalysatoren hydroformylierbar sind (vgl. J. Falbe, Ed.: New Syntheses With Carbon Monoxide, Springer, Berlin 1980, S. 55 ff), ist dieses Katalysatorsystem für interne und interne, verzweigte Olefine sowie für Olefine mit mehr als 7 Kohlenstoffatomen wenig geeignet (vgl. Falbe, l.c., S. 95 ff). So werden interne Kohlenstoff-Kohlenstoff-Doppelbindungen nur sehr langsam in Gegenwart eines derartigen Katalysators hydroformyliert. Da die Abtrennung des Hydroformylierungsproduktes von homogen in Reaktionssystem gelösten Katalysator in der Regel destillativ erfolgt und sich der Siedepunkt des bei der Hydroformylierung gebildeten Aldehyds mit zunehmender Kohlenstoffzahl und Kettenlänge auf Temperaturen erhöht, bei denen sich der rhodiumhaltige Katalysator zersetzt, ist diese Hydroformylierungsmethode für die Hydroformylierung von Olefinen mit mehr als 7 Kohlenstoffatomen nicht wirtschaftlich. Bei der Hydroformylierung polymerer Olefine, z.B. von Polyisobuten, kann der edelmetallhaltige Katalysator nicht in wiederverwendbarer Form wiedergewonnen werden.

Hingegen lassen sich interne und interne, verzweigte Olefine vorteilhaft mit sogenanntem "nacktem" Rhodium hydroformylieren, d.h. mit homogen im Hydroformylierungsmedium gelösten Rhodiumverbindungen, die nicht mit phosphorhaltigen Liganden, wie Phosphinen oder Phosphiten, modifiziert sind. Solche nicht mit Phosphinen oder Phosphiten modifizierte Rhodium-Katalysatoren und deren Eignung als Katalysator zur Hydroformylierung der zuvor genannten Klassen von Olefinen sind bekannt (siehe Falbe, l.c., S. 38 ff). Die Begriffe "nacktes Rhodium" oder "nackte Rhodium-Katalysatoren" werden im folgenden für Rhodium-Hydroformylierungskatalysatoren gebraucht, die im Gegensatz zu herkömmlichen Rhodium-Hydroformylierungskatalysatoren unter den Bedingungen der Hydroformylierung nicht mit Liganden, insbesondere nicht mit phosphorhaltigen Liganden, wie Phosphin- oder Phosphit-Liganden modifiziert sind. Als Liganden in diesem Sinn werden nicht Carbonyloder Hydrido-Liganden verstanden. Es wird in der Fachliteratur (s.Falbe, l.c., S. 38 ff) angenommen, daß die Rhodium-Verbindung HRh(CO)₄ die katalytisch aktiv Rhodiumspezies bei der Hydroformylierung mit "nackten Rhodium-Katalysatoren" ist, obgleich dies aufgrund der vielen in der Hydroformylierungszone nebeneinander ablaufenden Chemismen nicht eindeutig bewiesen ist. Lediglich der Einfachheit halber wird auch im folgenden von dieser Annahme Gebrauch, ohne daß hier als eine Beschränkung gelten soll, falls sich zukünftig einmal eine andere Rhodiumspezies als die genannte, als die eigentliche katalytisch aktive, herausstellen sollte. Die "nackten Rhodium-Katalysatoren" bilden sich unter den Bedingungen der Hydroformylierungsreaktion aus Rhodium-Verbindungen, z.B. Rhodiumsalzen, wie Rhodium(III)chlorid, Rhodium(III)nitrat, Rhodium(III)acetat, Rhodium(III)acetylacetonat, Rhodium(III)sulfat oder Rhodium(III)ammoniumchlorid, aus Rhodiumchalkogeniden, wie Rhodium(III)oxid oder Rhodium(III)sulfid, aus Salzen von Rhodiumsauerstoffsäuren, beispielsweise den Rhodaten, aus Rhodium-Carbonylverbindungen, wie Rhodiumdicarbonylacetylacetonat, Cyclooctadien-Rhodium-acetat oder -chlorid in Gegenwart von CO/H₂-Gemischen, die gemeinhin als Synthesegas bezeichnet werden. Zur Durchführung von Hydroformylierungen mit "nacktem" Rhodium sei an dieser Stelle beispielhaft auf die folgende Vorliteratur verwiesen: US-A 4 400 547; DE-A 33 38 340; DE-A 26 04 545; WO 82/03856; Chem. Ber. 102, 2238 (1969); Tetrahedron Lett. 29, 3261 (1968) und Hydrocarbon Process. 85-86 (1975).

Allerdings hat auch die Hydroformylierung mit "nacktem" Rhodium den Nachteil, daß sich der thermolabile Rhodium-Katalysator (vgl. US-A 4 400 547) infolge der thermischen Belastung bei der destillativen Aufarbeitung des Hydroformylierungsproduktes teilweise zu metallischem Rhodium zersetzt, das sich an den Wandungen des Reaktors und Rohrleitungen abscheidet. Das ausgefallene Rhodiummetall kann nicht wieder in die Hydroformylierungsreaktion zurückgeführt werden, da es unter den Hydroformylierungsbedingungen nicht in die katalytisch aktive Rhodiumverbindung umgewandelt werden kann. Die aus diesem chemischen Verhalten der "nackten Rhodium-Katalysatoren" resultierenden Rhodiumverluste haben bislang eine größere industrielle Anwendung dieses Verfahrens verhindert.

In DE-A 33 338 340 und US-A 4 400 547 werden Verfahren zur Hydroformylierung mittels "nackter Rhodium-Katalysatoren" beschrieben, bei denen zur Verhinderung der Rhodiumabscheidungen dem Reaktionsaustrag der Hydroformylierung ein Phosphin oder Phosphit zugesetzt wird, welche den Rhodium-Katalysator durch Bildung von Phosophin- oder Phosphit-Komplexen von einer thermischen Zersetzung im Zuge er destillativen Aufarbeitung des Hydroformylierungsaustrags schützen. Nach Abschluß der Destillation wird der rhodiumhaltige Destillationssumpf mit einem Oxidationsmittel behandelt, wobei das Rhodium in katalytisch aktiver Form aus den betreffenden Phosphin- oder Phosphit-Komplexen freigesetzt wird und die Phosphin- oder Phosphitliganden zu den entsprechenden unter Hydroformylierungsbedingungen keine Rhodium-Komplexe bildenden Phosphinoxiden und Phosphaten oxidiert werden. Der oxidierte Destillationssumpf wird dann erneut als Katalysator zur Hydroformylierung eingesetzt. Die bei der Oxidation entstandenen oxidierten Phosphorverbindungen stören bei der Hydroformylierung in der Regel nicht, jedoch reichern sich verfahrensbedingt die oxidierten Phosphorverbindungen in diesem Hydroformylierungskreislauf an, weshalb ständig ein Teilstrom dieser Katalysatorlösung aus dem Hydroformylierungskreislauf ausgeschleust und durch frische Katalysatorlösung ergänzt werden muß. Die ausgeschleuste Katalysatorlösung muß einer gesonderten Prozedur zur Wiedergewinnung des darin enthaltenen Rhodiums unterzogen werden.

WO 82/03856 betrifft ein Verfahren zur Thermostabilisierung von unmodifiziertem, also "nackten Rhodium-Katalysatoren", in dem der Austrag aus der Hydroformylierungsreaktion mit einem sauerstoffhaltigen Gas behandelt wird, wodurch die gebildeten Aldehyde z.T. zu den entsprechenden Carbonsäuren oxidiert werden, welche mit dem Rhodium-Katalysator bei der destillativen Aufarbeitung thermostabile Rhodium-Carboxylate bilden, die wieder als Katalysatoren zur Hydroformylierung verwendet werden können. Nachteilig an diesem Verfahren ist die Verringerung der Ausbeute als Folge der partiellen Oxidation der Produktaldehyde zu Carbonsäuren. Darüber hinaus ist dieses Verfahren auf solche Hydroformylierungen beschränkt, bei denen destillierbare Produkte gebildet werden: So kann nach diesem Verfahren z.B. der Rhodium-Katalysator nicht vom Hydroformylierungsprodukt des Polyisobutens abgetrennt werden.

Hydroformylierungen mit u.a. Cobalt und Rhodium in Gegenwart von mehrzähnigen, stickstoffhaltigen Liganden mit mindestens zwei Stickstoffresten, wie Bipyridin und N,N,N',N'-Tetramethylethylendiamin, sind in US 3 594 425 beschrieben. Durch diese Liganden erhält man eine Stabilisierung des Katalysators während der destillativen Aufarbeitung und der anschließenden Katalysatorrückführung, der sogenannten "Sumpffahrweise". Nach Abschluß der Destillation wird der rhodiumhaltige Destillationssumpf erneut als Katalysator zur Hydroformylierung eingesetzt. Die bei der Hydroformylierung und destillativer Aufarbeitung entstandenen Hochsieder stören bei der Hydroformylierung in der Regel nicht, reichern sich jedoch verfahrensbedingt in diesem Hydroformylierungskreislauf an, weshalb ständig ein Teilstrom dieser Katalysatorlösung aus dem Hydroformylierungskreislauf ausgeschleust und durch frische Katalysatorlösung ergänzt werden muß. Die ausgeschleuste Katalysatorlösung muß einer gesonderten Prozedur zur Wiedergewinnung des darin enthaltenen Rhodiums unterzogen werden. Darüber hinaus ist dieses Verfahren auf solche Hydroformylierungen beschränkt, bei denen destillierbare Produkte gebildet werden: So kann nach diesem Verfahren z.B. der Rhodium-Katalysator nicht vom Hydroformylierungsprodukt des Polyisobutens abgetrennt werden.

Rhodiumkomplexe mit mehrzähnigen, stickstoffhaltigen Liganden werden ferner als Katalysatoren für Hydroformylierungen in JP-A 262 086 (1994) beschrieben. Eine hohe Selektivität zu verzweigten Produkten wurde als besondere Eigenschaft solcher Systeme angegeben.

Die Zufuhr von stickstoffhaltigen Liganden wie Bipyridin erst zu einem Oxoaustrag, mit dem Ziel, die Ausfällung von Rhodium während der destillativen Aufarbeitung zu verhindern, ist in US 4 298 499 beschrieben. Die Hydroformylierung selbst wurde in Gegenwart eines tertiären Amin durchgeführt, um gezielt Alkohole zu erhalten. Nach Extraktion des Amins soll die Zufuhr von u.a. stickstoffhaltigen Liganden eine Rhodiumabtrennung ohne Rhodiumabscheidung ermöglichen. Der Destillationssumpf wird dann erneut als Katalysator in die Hydroformylierungszone zurückgeführt. Dies hat jedoch den bereits genannten Nachteil einer "Sumpffahrweise", nämlich der Notwendigkeit der Ausschleusung eines Teils des Rückstands und Wiedergewinnung des Rhodiums durch Aufarbeitung.

Schließlich ist eine reversible Extraktion von tertiär aminsubstituierten Triarylphosphinen aus EP 621 075 bekannt. Insbesondere ist dort eine Extraktion mit Hilfe von kohlensäurehaltigen wäßrigen Lösungen beschrieben. Die Entspannung der Lösung lieferte wieder einen im organischen Medium löslichen Katalysator. Dieses Konzept der Verwendung von aminsubstituierten Arylphosphinen ist mit Nachteilen verbunden, da zum einen Nebenreaktionen wie die Oxidation von Triarylphosphin zu Triarylphosphinoxid mittels Rhodium-Katalysatoren in Gegenwart von CO₂ bekannt ist und zum anderen Ausbeuteverluste durch Doppelbindungsisomerisierung zu befürchten sind. Schließlich werden interne Doppelbindungen durch Rh/Phosphin nur sehr langsam hydroformyliert. Ferner sind die tertiär aminsubstituierten Phosphine nur schwer herzustellen.

Der vorliegenden Erfindung lag daher die Aufgabe zugrunde, ein Verfahren zur Herstellung von Aldehyden aus internen und/oder verzweigten Olefinen mit Hilfe "nackter Rhodium-Katalysatoren" zu finden, das nicht die geschilderten Nachteile hat, mit dem nämlich die Probleme der Abscheidung metallischen Rhodiums bei der destillativen Aufarbeitung des Hydroformylierungsproduktes sowie die Rhodium-Katalysator-Abtrennung von nicht destillierbaren Produktaldehyden oder von reaktionseigenen Hochsiedern zufriedenstellend gelöst werden können.

Diese Aufgabe wurde erfindungsgemäß gelöst mit einem Verfahren zur Herstellung von Aldehyden oder Aldehyden und Alkoholen durch Hydroformylierung von Olefinen mit mehr als 3 Kohlenstoffatomen, umfassend eine Hydroformylierungsstufe, in der man mittels eines in einem homogenen Reaktionsmedium gelösten Rhodiumkatalysators das Olefin bei 50 bis 1000 bar und einer Temperatur von 50 bis 180°C hydroformyliert und eine Katalysatorrückgewinnungsstufe durch Extraktion des Rhodiumkatalysators, bei dem man
(a) die Hydroformylierung in Gegenwart eines Rhodiumkomplexes durchführt, der als Ligand eine phosphorfreie, mehrzähnige, zur Komplexbildung mit Metallen der Gruppe VIII des periodischen Systems befähigte, organische Stickstoffverbindung aufweist, die zusätzlich mindestens einen, durch eine schwache Säure protonierbaren tertiären Stickstoffrest enthält,
(b) den Austrag der Hydroformylierungsstufe, gegebenenfalls nach Abtrennung oder teilweiser Abtrennung der Aldehyde und Alkohole einer Extraktion mit einer wäßrigen Lösung einer destillierbaren Säure unterwirft,
(c) das wäßrige Säureextrakt in Gegenwart eines organischen Lösungsmittels oder Lösungsmittelgemischs, das unter den Hydroformylierungsbedingungen inert ist, unter Abdestillieren der wäßrigen Säure einer Hitzebehandlung, durch die der Komplex entprotoniert und in die organische Phase überführt wird, unterwirft und
(d) die organische, den Katalysatorkomplex enthaltende Phase in die Hydroformylierungsstufe zurückführt.

Dieses neue Verfahren führt überraschenderweise zu ausgezeichneten Ergebnissen nicht nur in Bezug auf die einfache Möglichkeit der Katalysatorrückgewinnung, sondern auch wegen der hohen Ausbeuten an Aldehyden gegenüber Alkoholen auch bei niedrigen Hydroformylierungstemperaturen, wohingegen gemäß US 4 298 499 und Chem. Ing. Techn. 44, 708 (1972) zu erwarten gewesen wäre, daß die Aldehydausbeute in Gegenwart von tert. Amingruppen enthaltenden Liganden relativ gering und damit nicht ausreichend für ein großtechnisches Verfahren sein würde.

Erfindungsgemäß werden vor allem bifunktionelle mehrzähnige organische Stickstoffverbindungen als Liganden verwendet, die mindestens zwei Stickstoffatome aufweisen, die einen Komplex mit Übergangsmetallen der 8. Nebengruppe bilden können und zusätzlich mindestens eine tertiäre Aminogruppe enthalten, die protoniert werden kann.

Unter Einwirkung dieser Liganden bilden sich - vermutlich über die freie Elektronenpaare der Stickstoffatome - koordinative Bindungen mit dem Rhodium-Zentralatom des Rhodium-Katalysators aus. Solche Verbindungen werden Komplexbildner genannt. Die Anwesenheit von mindestens einer tertiären Aminogruppe, die protonierbar ist und nicht an das Rhodium-Zentralatom des Rhodium-Katalysators gebunden ist, ist kritisch für die Durchführbarkeit des erfindungsgemäßen Verfahrens.

Insbesondere werden die Verbindungen der Formel 1 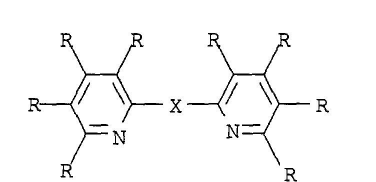 verwendet, in der X ein Brückenglied, ausgewählt aus der Gruppe, bestehend aus einer kovalenten Bindung, Methylen, Ethylen, Oxo, Thio, Alkylimino und Arylimino und der Reste R unabhängig voneinander Wasserstoff, Alkylreste mit 1 bis 18 C-Atomen oder Alkoxyreste mit 1 bis 18 C-Atomen bedeuten, wobei diese Reste Bestandteil eines gesättigten oder ungesättigten Rings sein können und wobei mindestens einer der Reste R oder gegebenenfalls der Substituenten an einem Ring, der durch die Alkyl- oder Alkoxyreste gebildet wird, ein Rest der Formel 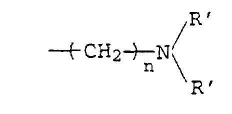 ist, in der n die Zahlen 0 bis 20 und R' Alkyl-, Cycloalkyl-, Aralkyl oder Arylreste mit bis zu 18 C-Atomen bedeutet, wobei die Reste R' miteinander verbrückt sein können.

Als Liganden kommen auch Polyamine der Formel 2 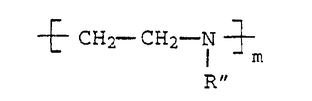 in Betracht, wobei R" Wasserstoff, Alkyl-, Cycloalkyl-, Aryl- oder Aralkylreste, die ihrerseits Dialkylaminoreste tragen können, bedeuten und m die Zahlen 2 bis 35000 bedeutet, mit der Maßgabe, daß im Falle der Bedeutung von R" = Wasserstoff mindestens ein Teil der Wasserstoffatome durch Alkylcarbonyl mit 2 bis 18 C-Atomen oder durch Hydroxy(alkoxy)-alkylreste, die durch Umsetzung der sekundären Aminogruppe mit 1 bis 10 Mol Ethylenoxid oder 1 bis 10 Mol Propylenoxid erhalten werden, substituiert sind und mit der weiteren Maßgabe, daß das Polyamin mindestens 3 tertiäre, zur Protonisierung befähigte Stickstoffatome enthält.

Besonders bevorzugt sind tertiäraminsubstituierte Bipyridine der Formel 3 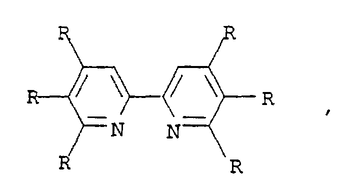 in der die Reste R die für Formel 1 angegebenen Bedeutungen haben und die mindestens einen eine tertiäre Aminogruppe enthaltenden Rest der Formel 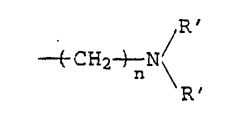 tragen, in der n und R' die oben angegebene Bedeutung haben.

Repräsentative Beispiele sind die Liganden 1 und 2 und deren Stellungsisomere 1' und 2' sowie durch weitere inerte Substituenten substituierte Verbindungen. 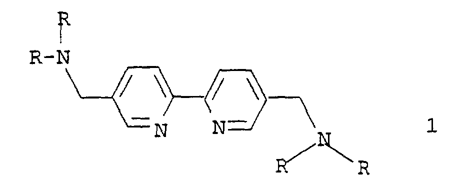 oder 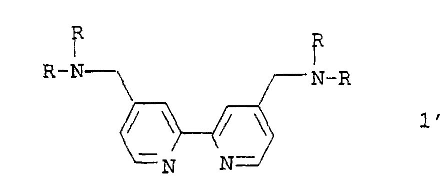 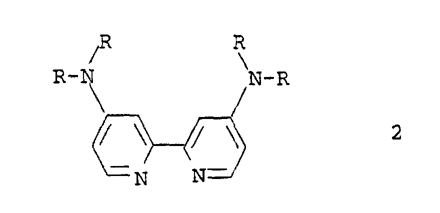 oder

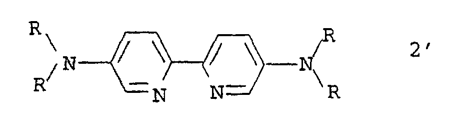

Zur Verdeutlichung der Vielzahl der erfindungsgemäß zu verwenden den Liganden werden im folgenden beispielhaft eine Reihe von wei teren stickstoffhaltigen Komplexbildnern genannt, die nach Einführung einer zusätzlichen tertiären Aminogruppe im erfindungsgemäßen Verfahren eingesetzt werden können.
2,2'-Bichinoline wie z.B. Ligand 3 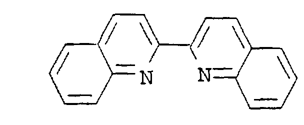 5,6,5',6'-Dibenzo-2,2'-bichinoline wie z.B. Ligand 4 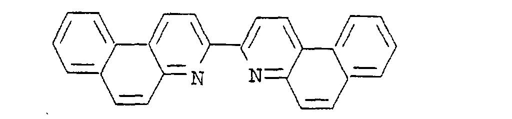 1,10-Phenanthroline, 2,9-Dimethylphenanthroline,
4,7-Diphenyl-1,10-phenanthronline wie z.B. Ligand 5 ("Bathophenanthrolin") 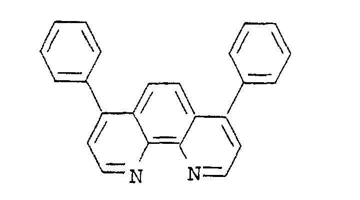 2,9-Dimethyl-4,7-diphenyl-1,10-phenanthroline wie z.B. Ligand 6 ("Bathocuproin") 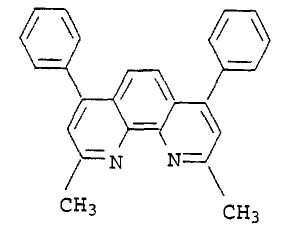 4,5-Diazofluorene wie z.B. Ligand 7 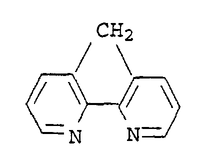 Dipyrido [3,2-a:2',3'-c)phenazine wie z.B. Ligand 8 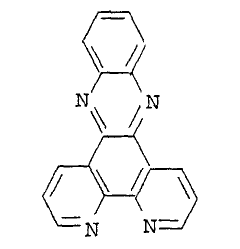 (Liganden 7 und 8 sind erhältlich nach Aust. J. Chem., 23, 1023 (1970))
2,2',6',2"-Terpyridine wie z.B. Ligand 9 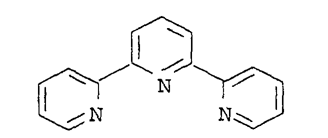 4'-Phenyl-2,2',6',2"-terpyridine wie z.B. Ligand 10 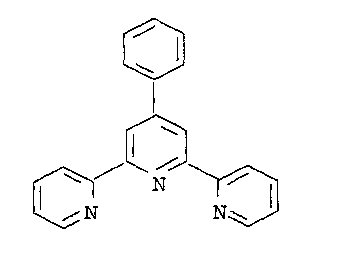 4-Methyl-(4'-phenyl)-(4"-methyl)-2,2',6',2"-terpyridine wie z.B. Ligand 11

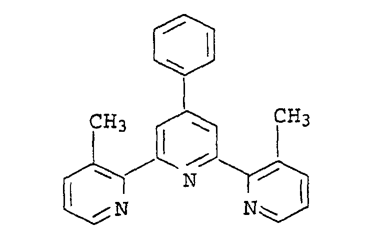

Weitere geeignete mehrzähnige Stickstoff enthaltende Liganden sind Bipyrrole, Bipyrazole, Bisimidazole, Bitriazole, Bitetrazole, Bipyridazine, Bipyrimidine, Bipyrazine, Bitriazine sowie die Porphine. Die Liganden können auch unsymmetrisch aufgebaut sein, beispielsweise durch Kopplung eines Imidazols mit einem Pyridin.

Die Hydroformylierung mit dem Rhodiumkomplex mit den mehrzähnigen stickstoffhaltigen Liganden erfolgt unter an sich bekannten Bedingungen. Sie wird im allgemeinen bei Temperaturen von 60 bis 180°C, vorzugsweise 80 bis 140°C und besonders bevorzugt bei 90 bis 130°C und bei einem Druck von im allgemeinen 50 bis 1000 bar, vorzugsweise bei 70 bis 500 bar, insbesondere bei 100 bis 400 bar durchgeführt. Die Hydroformylierung erfolgt ansonsten unter Bedingungen, wie sie üblicherweise bei Hydroformylierungen mit "nacktem" Rhodium angewandt werden und wie sie beispielsweise in der eingangs zitierten Literatur, betreffend die Hydroformylierung mit "nacktem" Rhodium, beschrieben sind.

In Abhängigkeit von den in der Hydroformylierungsstufe angewandten Druck- und Temperaturbedingungen und der Synthesegaszusammensetzung kann das Produktverhältnis Alkohol/Aldehyd im Hydroformylierungsaustrag beeinflußt werden. Beispielsweise wird bei der Hydroformylierung von Trimerpropylen bei jeweils gleichen Synthesegaszusammensetzungen - CO/H₂-Molverhältnis 50:50, 40:60 bzw. 60:40 - bei 130°C und einem Druck von 280 bar ein Aldehyd/Alkohol-Molverhältnis von jeweils 93:7 erhalten. Bei Erhöhung der Temperatur von 130°C auf 150°C verändert sich das Aldehyd/Alkohol-Molverhältnis im Hydroformylierungsaustrag in Abhängigkeit von der Synthesegaszusammensetzung - CO/H₂-Molverhältnis 50:50, 40:60 bzw. 60:40 auf 76:24, 67:33 bzw. 82:18.

Die Hydroformylierung kann in An- oder Abwesenheit von organischen Lösungsmitteln durchgeführt werden. Besonders vorteilhaft ist die Verwendung organischer Lösungsmittel, insbesondere bei der Hydroformylierung langkettiger oder polymerer Olefine. Als Lösungsmittel können die im Hydroformylierungsverfahren üblicherweise eingesetzten Lösungsmittel benutzt werden, beispielsweise hochsiedende aromatische und aliphatische Kohlenwasserstoffe oder auch hochsiedende Aldehyd-Kondensationsprodukte, die im Zuge der Hydroformylierungsreaktion als Nebenprodukt als Folge der Kondensation der Produktaldehyde entstehen.

Der Austrag aus der Hydroformylierungsstufe wird vor seiner Extraktion mit der wäßrigen sauren Phase zweckmäßigerweise entspannt. Die Extraktion des Hydroformylierungsaustrags wird im allgemeinen bei Temperaturen von 20 bis 140°C, vorzugsweise von 70 bis 130°C, insbesondere von 90 bis 120°C und bei einem Druck von im allgemeinen 1 bis 20 bar, vorzugsweise 1 bis 10 bar und besonders bevorzugt von 1 bis 5 bar durchgeführt. Die Extraktion kann an der Luft oder unter einer Inertgasatmosphäre durchgeführt werden, beispielsweise einer Stickstoff-, Wasserstoff- oder Argonatmosphäre, es kann aber auch vorteilhaft sein, dem verwendeten Inertgas zusätzlich Kohlenmonoxid oder Synthesegas beizumischen oder die Extraktion in Gegenwart von Kohlenmonoxid durchzuführen.

Bei der Extraktion wird im allgemeinen ein Volumenverhältnis wäßrige/organische Phase von im allgemeinen 0,2 bis 2, vorzugsweise von 0,3 bis 1, eingestellt. Der Gehalt der wäßrigen sauren Phase an wasserlöslichen, schwachen Säuren beträgt im allgemeinen 0,1 bis 50 %, bevorzugt 1 bis 30 % und besonders bevorzugt 3 bis 10 %.

Bei der Extraktion mit der sauren wäßrigen Phase findet eine Protonierung an der tertiären Aminogruppe des Liganden statt, wodurch die Rhodiumkomplexe wasserlöslich werden.

Als Extraktionsmittel für das erfindungsgemäße Verfahren verwendet man wäßrige Lösungen von destillierbaren Säuren, insbesondere destillierbare Säuren, deren Ammoniumsalze zwischen Raumtemperatur und 200°C in freien Ligand und freie Säure zurückgespalten werden können.

Insbesondere kommen als Extraktionsmittel wäßrige Lösungen von Säuren in Betracht, deren pKₛ-Wert 3 bis 6 beträgt. Im einzelnen sind Kohlensäure, Ameisensäure, Essigsäure, Propionsäure, n-Buttersäure oder Valeriansäure zu nennen. Im Falle von Kohlensäure wird dabei die zu extrahierende, den Katalysator enthaltende organische Flüssigkeit wie in EP 0 621 075 beschrieben mit Wasser versetzt, Kohlensäuregas aufgepreßt und nach Phasentrennung wie bei den anderen Säuren beschrieben weiterverarbeitet.

Zur Extraktion des Hydroformylierungsaustrags mit der wäßrigen sauren Phase sind praktisch alle flüssig-flüssig-Extraktionsapparaturen geeignet, beispielsweise Mixer-Settler-Apparaturen, Blasensäulen oder Gegen- oder Gleichstromextraktionskolonnen, wobei diese noch mit zusätzlichen Einbauten zur besseren Durchmischung von wäßriger und organischer Phase versehen sein können, beispielsweise mit Siebböden, Füllkörpern oder statischen Mischern. Die Extraktion des Rhodium-Katalysators aus dem Hydroformylierungsaustrag kann einstufig durchgeführt werden, vorzugsweise wird eine Mehrstufenextraktion angewandt, beispielsweise eine Zwei- oder Dreistufenextraktion, wobei die wäßrige, den Komplexbildner enthaltende Phase im Gleichstrom oder besonders bevorzugt, im Gegenstrom bezüglich der organischen Phase geführt werden kann.

Nach beendeter Extraktion kann der vom Rhodium-Katalysator befreite Hydroformylierungsaustrag auf an sich herkömmliche Weise, beispielsweise destillativ zur Isolierung der darin enthaltenen Wertprodukte-Alkohole und/oder -Aldehyde aufgearbeitet werden.

Zur Freisetzung der in dem wäßrigen Extrakt gelösten Rhodiumkomplexe und Überführung in eine organische, in die Hydroformylierung zurückführbare Phase wird das wäßrige saure Extrakt mit einem für die Hydroformylierung geeigneten Lösungsmittel, z.B. einem flüssigen Kohlenwasserstoff, oder einem Kohlenwasserstoffgemisch z.B. Texanol® der Firma Eastman versetzt. Als Lösungsmittel geeignete Produkte der Hydroformylierung oder Nebenprodukte der Hydroformylierung sind gleichermaßen geeignet. Das Volumenverhältnis des Lösungsmittels zu wäßriger Phase beträgt dabei in der Regel 0,1 bis 2, vorzugsweise 0,5 bis 1.

Das Gemisch aus Lösungsmittel und wäßrig saurer Phase wird dann auf solche Temperaturen erhitzt, daß das Ammoniumsalz der schwachen Säure gespalten und der entprotonierte Rhodiumkomplex im Lösungsmittel gelöst wird. Nach Trennung der organischen von der wäßrigen Phase, wenn vorhanden, wird die den Rhodiumkatalysator nun enthaltende organische Phase in die Hydroformylierung zurückgeführt.

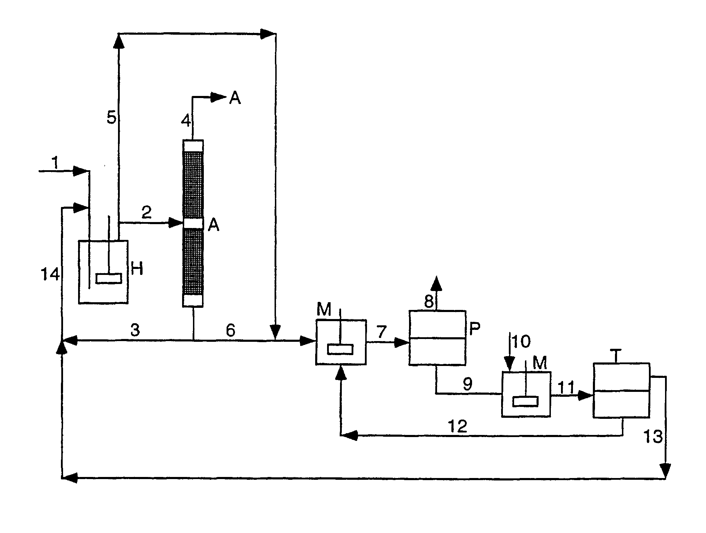

Die Entprotonierung erfolgt in der Regel unter Durchmischung der organischen und wäßrigen Phase und Erhitzen auf z.B. bis 90°C für die Entfernung von CO₂ bzw. auf z.B. bis 150°C für die Entfernung der Carbonsäuren, wobei eine Temperatur von 200°C im allgemeinen nicht überschritten zu werden braucht.
Nach einer vorteilhaften Ausgestaltung des erfindungsgemäßen Verfahrens wie sie in Figur 1 schematisch dargestellt ist, wird z.B. im einzelnen verfahren. An sich selbstverständliche Anlagendetails, die zu der Veranschaulichung des erfindungsgemäßen Verfahrens nicht erforderlich sind, sind dabei aus Gründen der Übersichtlichkeit in Figur 1 nicht enthalten.

Die in Figur 1 dargestellte Ausgestaltung des erfindungsgemäßen Verfahrens umfaßt die Verfahrensstufen der Hydroformylierung mittels eines homogen im organischen Reaktionsmedium gelösten Rhodium-Katalysators, der Abtrennung des Katalysators vom Austrag der Hydroformylierungsreaktion und der Rückführung des vom Hydroformylierungsaustrag abgetrennten Rhodiums in die Hydroformylierungsstufe. Die Abtrennung des Katalysators erfolgt gegebenenfalls nach teilweiser oder vollständiger Destillation durch reversible Extraktion des Katalysators aus dem Reaktoraustrag oder Destillationssumpf mit einer sauren, wäßrigen Phase. Durch anschließende thermische Spaltung des Säure-Ligandaddukts nach Extraktion wird der Katalysator wieder in eine organische Phase überführt und damit die Rückgewinnung des Rhodiums ohne Salzanfall gewährleistet. Anschließend erfolgt die Rückführung des Rhodiums in die Hydroformylierungsstufe. Bei der kontinuierlichen Ausgestaltung wird zweckmäßigerweise solange allein eine destillative Katalysatorabtrennung durchgeführt bis die Anreicherung von Nebenprodukten eine Extraktion erforderlich macht.

In einer kontinuierlichen Ausgestaltung des erfindungsgemäßen Verfahrens gemäß Figur 1 wird im einzelnen der Hydroformylierungsaustrag des hydroformylierten Olefins 1 aus dem Hydroformylierungsreaktor H nach Entspannung und Abtrennung der flüssigen Phase von überschüssigem Synthesegas wahlweise über Leitung 5 einer extraktiven Aufarbeitung oder zunächst über Leitung 2 einer destillativen Aufarbeitung (bestehend aus Flash und/oder Kolonne) zugeführt. In der destillativen Aufarbeitung A werden die leichtersiedenden Aldehyde 4 von einem hochsiedenden katalysatorhaltigen Sumpf getrennt. Der Sumpf wird über Leitung 3 zeitweise oder teilweise in die Hydroformylierungsstufe zurückgeführt und zeitweise oder teilweise über Leitung 6 einer extraktiven Aufarbeitung zugeführt. In der extraktiven Aufarbeitung wird in einem Mixer M eine säurehaltige Wasserphase mit der organischen Phase gemischt. Diese Mischung wird über Leitung 7 einer Phasentrennung P zugeführt. Die organische Phase, bestehend aus Aldehyden und/oder hochsiedenden Komponenten, wird über Leitung 8 abgetrennt und steht für weitere Verarbeitung zur Verfügung. Die wäßrige Phase wird über Leitung 9 in einen Mixer M geleitet und mit einer mit der Hydroformylierung verträglichen organischen Phase 10 z.B. Olefinen, Aldehyden und/oder Alkoholen, Toluol oder anderen Aromaten oder Gemischen wie Texanol® oder Ethern wie Diethylether gemischt. Diese Mischung wird über Leitung 11 einer thermischen Spaltung T zugeführt. Diese kann z.B. eine Destillationskolonne sein, um das Wasser/Carbonsäuregemisch abzutrennen, oder allein eine thermische Behandlung, um CO₂ abzuspalten. Die wäßrige Phase kann wahlweise über Leitung 12 in die Extraktion zurückgeführt oder ausgeschleust werden. Die katalysatorhaltige organische Phase wird über Leitung 13 in der Hydroformylierungsstufe zurückgeführt.

Das erfindungsgemäße Verfahren eignet sich besonders gut zur Hydroformylierung von Olefinen mit mehr als 3, vorzugsweise mehr als 7 Kohlenstoffatomen, insbesondere zur Hydroformylierung von C₇- bis C₂₀-Olefinen, die geradkettig oder vor allem verzweigt sein können und die α-olefinische und/oder auch interne Doppelbindungen enthalten können, z.B. Octen-1, Dodecen-1, Trimer- und Tetramerpropylen, oder Dimer-, Trimer- und Tetramerbutylen. Ebenso können ungesättigte Oligomere anderer Olefine hydroformyliert werden, desgleichen Cooligomere verschiedener Oletine. Die aus diesen Olefinen gebildeten Aldehyde dienen z.B. als Vorstufen für die Herstellung von Weichmacheralkoholen und Tensiden, die daraus auf an sich bekannte Weise durch Hydrierung erzeugt werden können. Die zur Hydroformylierung eingesetzten Olefine können z.B. durch die säurekatalysierte Wasserabspaltung aus den entsprechenden Fettalkoholen oder nach einer Vielzahl anderer technischer Verfahren, wie sie beispielsweise in Weissermel, Arpe: Industrielle Organische Chemie, S. 67-86, Verlag Chemie, Weinheim, 1978, dargestellt sind, erhalten werden.

Besonders gut geeignet ist das erfindungsgemäße Verfahren auch zur Hydroformylierung von polymeren Olefinen, beispielsweise niedermolekularem Polyisobuten, niedermolekularem Polybutadien oder niedermolekularem 1,3-Butadien-Isobuten- oder Buten-Copolymeren. Unter "niedermolekulare Polymere" werden insbesondere Polymere mit Molgewichten von 280 bis 5000 Dalton verstanden. Es können aber auch höhermolekulare, ungesättigte Polymere mit Molgewichten von größer 5000 hydroformyliert werden. Einzige Voraussetzung hierfür ist, daß diese im Hydroformylierungsmedium löslich sind.

Das vorliegende Verfahren eignet sich demgemäß zur Herstellung praktisch aller Aldehyde, die auf dem Wege der Hydroformylierung von Olefinen erhältlich sind. Insbesondere können beispielsweise auch substituierte Olefine, die im allgemeinen 1 bis 2, vorzugsweise einen Substituenten tragen können, nach dem erfindungsgemäßen Verfahren hydroformyliert werden. Beispielsweise können nach dem erfindungsgemäßen Verfahren ungesättigte, aliphatische Carbonsäureester, Acetale, Alkohole, Ether, Aldehyde, Ketone und Amine und Amide hydroformyliert werden. Als derartige substituierte Ausgangsolefine sind z.B. Methacrylsäureester, Dicyclopentadien, Vinyl- und Allylether, insbesondere entsprechend substituierte Derivate ungesättigter Fettsäuren von Interesse, beispielsweise die Ester der Öl-, Linol-, Linolen-, Ricinol- oder Erucasäure. Die aus diesen olefinischen Rohstoffen durch Hydroformylierung erhältlichen Aldehyde sind ebenfalls Ausgangsmaterialien zur Herstellung biologisch leicht abbaubarer, waschaktiver Substanzen.

### Beispiele

A) Katalysatorprecursorsynthesen: 5,5'-Bis(dimethylaminomethyl)-2,2'-Bipyridin (Ligand 1) wird in drei Stufen aus ß-Picolin hergestellt:
   1. Stufe:
      Die oxidative Verknüpfung von 4-Picolin zu 4,4'-Dimethyl-2,2'-bipyridin ist in J. Chem. Soc., Dalton Trans., 1985, S. 2247 beschrieben. Die Verknüpfung von β-Picolin zu 5,5'-Dimethyl-2,2'-bipyridin läuft völlig analog ab und wurde wie dort beschreiben durchgeführt. 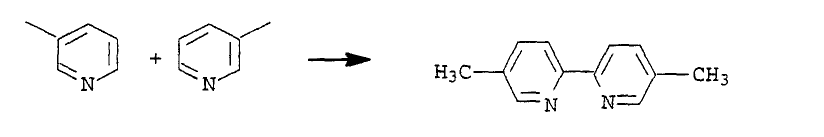
   2. Stufe:
      Die radikalische Bromierung von 6,6'-Dimethyl-2,2'-bipyridin in CCl₄ in Gegenwart von einem Radikalstarter wie Benzoylperoxid ist in Helv. Chim. Acta, Vol 67, S. 2264, 1984 beschrieben. Die Bromierung von 5,5'-Dimethyl-2,2'-bipyridin in Gegenwart von 2,2-Azoisobuttersäurenitril als Radikalstarter mit N-Bromsuccinimid läuft völlig analog ab und wurde wie dort beschrieben durchgeführt. 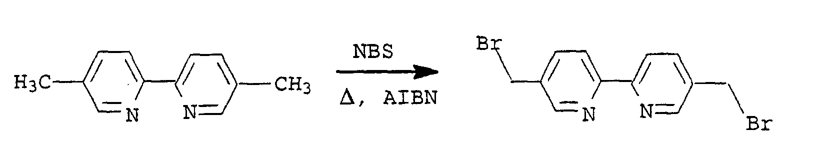
   3. Stufe:
      Anschließend wurde 5,5'-Bis (brommethyl)-2,2'-bipyridin mit LiNMe₂ in THF zu 5,5'-Bis(dimethylaminomethyl)-2,2'-bipyridin umgesetzt. Die Lösung wurde mit einer 1 %igen NaHCO₃-Lösung gequencht und Ethylether zugegeben bis sich eine zweite Phase gebildet hat. Die Aufarbeitung der organischen Phase lieferte den Ligand 1. 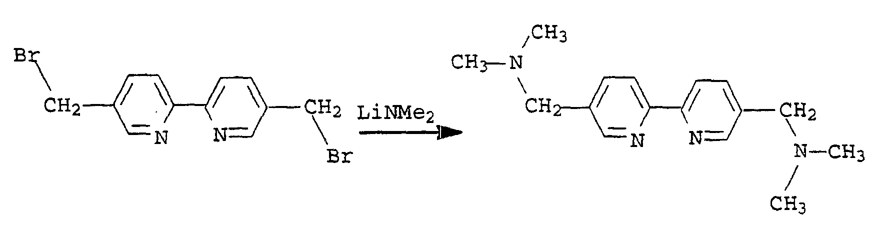 Alternativ und in Analogie zu Stufe. 1 ist auch eine Verknüpfung von 4-Dimethylaminopyridin zu 4,4'-Bis(dimethylamino)-2,2'-bipyridin möglich. 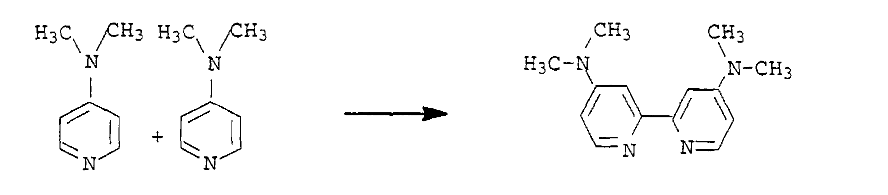
B) Allgemeine Bedingungen einer diskontinuierlichen Verfahrensführung:
   Alle diskontinuierlichen Hydroformylierungen bei 70 bar wurden in einem 100 ml Autoklav (Material HC) durchgeführt. Das Reaktionsgemisch wurde 10 min lang auf Reaktionstemperatur aufgeheizt, wobei die Lösung mit einem Begasungsrührer kräftig gerührt wurde. Alle diskontinuierlichen Hydroformylierungen bei 280 bar wurden in einem 300 ml Autoklav (Material HC) durchgeführt. Das Reaktionsgemisch wurde 45 min lang auf Reaktionstemperatur aufgeheizt und die Lösung wurde mit einem Magnetrührer kräftig gerührt. Mittels CO/H₂ (1:1) wurde in beiden Fällen der gewünschte Druck eingestellt, wobei während der Reaktion der Druck im Reaktor durch Nachpressen über einen Druckregler auf Druckniveau gehalten wurde. Nach der Reaktionszeit wurde der Autoklav abgekühlt, entspannt und entleert. Eine Analyse des Reaktionsgemisches wurde mittels GC - mit internem Standard und Korrekturfaktoren - durchgeführt.
   Beispiel 1) Hydroformylierung mit Rh/Ligand 1 sowie Abtrennung durch Extraktion mit Essigsäurelösungen und Katalysatorrückführung
   a) Bei der an sich bekannten Hydroformylierung von Octen-N (Isooctengemisch, Verzweigungsgrad 1,3; 98 g) mit Rhodium/Ligand 1 (13 ppm Rh, L/Rh = 10, 150°C, 280 bar CO/H₂ 1:1, 5 h, mit 5 g Texanol® als Lösungsmittel) wurde ein Umsatz der Octene von 98 %, eine Ausbeute an Nonanal von 76 % und Nonanol von 19 % bei 3 %igem Bilanzverlust (bez. auf Octen-N) erhalten.
   b) Destillative Abtrennung der Oxoprodukte bei 20 mbar bis zu 150°C Sumpftemperatur.
   c) Wiederholung des Schritts 1 (12 ppm Rh), wobei man Rhodiumkatalysator/Sumpflösung aus (b) einsetzt. Ein Umsatz an Octenen von 99 %, eine Ausbeute an Nonanal von 66 % und Nonanol von 15 % wurde bei 16 %igem Bilanzverlust (bez. auf Octen-N; mechanische Verluste, Hochsieder- und Paraffinbildung mitbeinhaltet) erhalten.
   d) Destillative Abtrennung der Oxoprodukte (99 g) bei 20 mbar bis zu 150°C Sumpftemperatur (16 g), Extraktion des Sumpfes mit 5 %iger Essigsäure (1:1 vol:vol)) und anschließende Phasentrennung. Die Phasentrennung wurde durch Filtration über ein Kieselguhrbett und Papierfilter beschleunigt. 15 ppm Rh wurden in der organischen Phase gefunden, d.h. Rhodium wurde mit 80 %igem Effizienz extrahiert. Nach Zufuhr von Texanol® zur wäßrigen Phase und anschließende Verdampfung von Essigsäure/Wasser unter Normaldruck bis zu 150°C Sumpftemperatur wurde eine homogene Lösung erhalten.
   e) Wiederholung des Schritts 1 (12 ppm Rh), wobei man Rhodiumkatalysator/Texanol®-Lösung aus (d) einsetzte. Ein Octenumsatz von 97 %, eine Ausbeute an Nonanal von 67 % und eine Ausbeute an Nonanol von 10,5 % wurde bei 19 %igem Bilanzverlust (bez. auf Octen-N) erreicht. Eine direkte Hochsiederbestimmung durch destillative Abtrennung der Oxoprodukte bei 150°C bei 20 mbar ergab nur 5 % Hochsieder.
   f) Bei unmittelbarer Extraktion des Reaktoraustrags mit 5 %iger Essigsäure (3 x 50 ml) und anschließende Phasentrennung wurden 3 ppm Rh in der organischen Phase gefunden, d.h. Rhodium wurde mit 94 %igem Effizienz extrahiert. Durch Zufuhr von Texanol® zur wäßrigen Phase und anschließende destillative Aufarbeitung unter Normaldruck bis zu 150°C Sumpftemperatur wurde der Ligand entprotoniert.
   g) Die Hydroformylierung von C₁₂- bis C₁₄-α-Olefingemisch (94 g) mit Rhodiumkatalysator/Texanol® -Lösung aus (f) (2 ppm Rh, 100°C, 280 bar CO/H₂ 1:1, 4 h) ergab einen Olefinumsatz von 98 %, eine Aldehydausbeute von 81 % und eine Selektivität für lineare Isomere von 47 % bei 6,5 %igem Bilanzverlust (bez. auf α-Olefine; mechanische Verluste, Hochsieder- und Paraffinbildung mitbeinhaltet) eingeschlossen.

### Beispiel 2) Hydroformylierung mit Rh/Ligand 1 sowie Abtrennung durch Extraktion mit CO₂/H₂O-Lösung

a) Die Hydroformylierung von Octen-N (Isooctengemisch, Verzweigungsgrad 1,3; 95 g) mit Rhodium/Ligand 1 (13 ppm Rh, L/Rh = 10, 150°C, 280 bar CO/H₂ 1:1 während 5 h in 5 g Texanol® als Lösungsmittel) ergab einen Octenumsatz von 98 %, eine Ausbeute an Nonanal + Nonanol von 79 % und einen 18 %igen Bilanzverlust (bez. auf Octen-N).
b) Bei Extraktion des Reaktoraustrags (23 g) aus 1 unter Zusatz von 20 g Wasser durch Aufpressen von CO₂ unter einem Druck von einem bar CO₂ und anschließende Phasentrennung wurden 4 ppm Rh in der organischen Phase und 7 ppm in der wäßrigen Phase gefunden. Rhodium wurde mit 85 %igem Effizienz extrahiert.
c) Die Rückführung des Rhodiumkomplexes erfolgte wie in Beispiel 1 beschrieben.

## Patentansprüche

1. Verfahren zur Herstellung von Aldehyden oder Aldehyden und Alkoholen durch Hydroformylierung von Olefinen mit mehr als 3 Kohlenstoffatomen umfassend eine Hydroformylierungsstufe, in der man mittels eines in einem homogen Reaktionsmedium gelösten Rhodiumkatalysators das Olefin bei 50 bis 1000 bar und einer Temperatur von 50 bis 180°C hydroformyliert und eine Katalysatorrückgewinnungsstufe durch Extraktion des Rhodium-katalysators, dadurch gekennzeichnet, daß man
(a) die Hydroformylierung in Gegenwart eines Rhodiumkomplexes durchführt, der als Ligand eine phosphorfreie, mehrzähnige, zur Komplexbildung mit Metallen der Gruppe VIII des periodischen Systems befähigte, organische Stickstoffverbindung aufweist, die zusätzlich mindestens einen, durch eine schwache Säure protonierbaren tertiären Stickstoffrest enthält,
(b) den Austrag der Hydroformylierungsstufe, gegebenenfalls nach Abtrennung oder teilweiser Abtrennung der Aldehyde und Alkohole einer Extraktion mit einer wäßrigen Lösung einer destillierbaren Säure unterwirft,
(c) das wäßrige Säureextrakt in Gegenwart eines organischen Lösungsmittels oder Lösungsmittelgemischs, das unter den Hydroformylierungsbedingungen inert ist, unter Abdestillieren der wäßrigen Säure einer Hitzebehandlung, durch die der Komplex entprotoniert und in die organische Phase überführt wird, unterwirft und
(d) die organische, den Katalysatorkomplex enthaltende Phase in die Hydroformylierungsstufe zurückführt.

2. Verfahren gemäß Anspruch 1, dadurch gekennzeichnet, daß man destillierbare Säuren verwendet, deren Ammoniumsalze zwischen Raumtemperatur und 200°C in freien Ligand und freie Säure zurückgespalten werden können.

3. Verfahren gemäß Anspruch 1, dadurch gekennzeichnet, daß man Liganden der Formel I 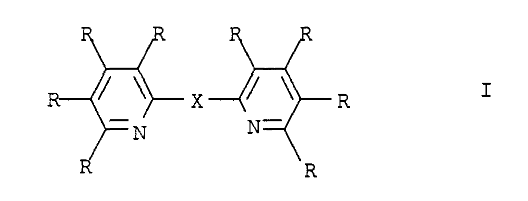 verwendet, in der X ein Brückenglied, ausgewählt aus der Gruppe, bestehend aus einer kovalenten Bindung, Methylen, Ethylen, Oxo, Thio, Alkylimino und Arylimino und die Reste R unabhängig voneinander Wasserstoff, Alkylreste mit 1 bis 18 C-Atomen oder Alkoxyreste mit 1 bis 18 C-Atomen bedeuten, wobei diese Reste Bestandteil eines gesättigten oder ungesättigten Rings sein können und wobei mindestens einer der Reste R oder gegebenenfalls der Substituenten an einem Ring, der durch die Alkyl- oder Alkoxyreste gebildet wird, ein Rest der Formel II 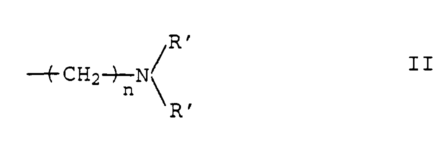 ist,
in der n die Zahlen 0 bis 20 und R' Alkyl-, Cycloalkyl-, Aralkyl oder Arylreste mit bis zu 18 C-Atomen bedeutet, wobei die Reste R' miteinander verbrückt sein können.

4. Verfahren gemäß Anspruch 1, dadurch gekennzeichnet, daß man als Liganden Polyamine der Formel III 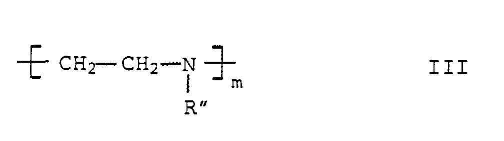 verwendet, in der R" Wasserstoff, Alkyl-, Cycloalkyl-, Aryl- oder Aralkylreste, die ihrerseits Dialkylaminoreste tragen können, bedeuten und m die Zahlen 2 bis 35 000 bedeutet, mit der Maßgabe, daß im Falle der Bedeutung von R" = Wasserstoff mindestens ein Teil der Wasserstoffatome durch Alkylcarbonyl mit 2 bis 18 C-Atomen oder durch Hydroxy(alkoxy)-alkylreste, die durch Umsetzung der sekundären Aminogruppe mit 1 bis 10 Mol Ethylenoxid oder 1 bis 10 Mol Propylenoxid erhalten werden, substituiert sind und mit der weiteren Maßgabe, daß das Polyamin mindestens 3 tertiäre, zur Protonisierung befähigte Stickstoffatome enthält.

5. Verfahren gemäß Anspruch 1, dadurch gekennzeichnet, daß man als Liganden Bipyridine der Formel IV 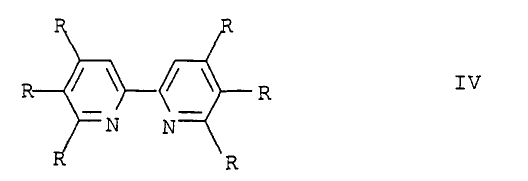 verwendet, in der die Reste R die in Anspruch 3 angegebenen Bedeutungen haben und die mindestens einen eine tertiäre Aminogruppe enthaltenden Rest der Formel II 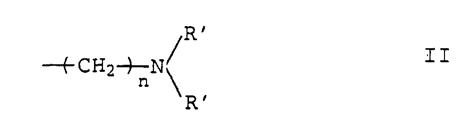 darstellen, bzw.
tragen, in der n und R' die in Anspruch 3 angegebene Bedeutung hat.

6. Verfahren gemäß Anspruch 1, dadurch gekennzeichnet, daß man als Liganden Verbindungen der Formeln 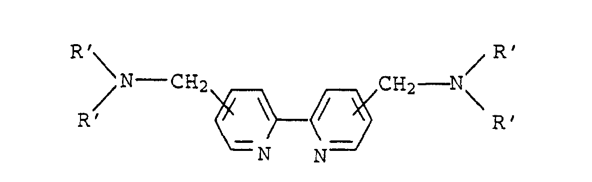 verwendet, in der der Rest 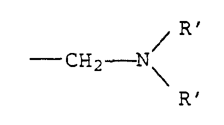 in 4,4'- oder in 5,5'-Stellung an die Pyridylringe gebunden ist und in der R' unabhängig voneinander Alkyl-, Cycloalkyl, Aralkyl- oder Arylreste mit bis zu 18 C-Atomen bedeutet.

7. Verfahren gemäß Anspruch 1, dadurch gekennzeichnet, daß man zur Extraktion Säuren mit pK-Werten von 3 bis 6 verwendet.

8. Verfahren gemäß Anspruch 1, dadurch gekennzeichnet, daß man zur Extraktion wäßrige Lösungen von Kohlensäure, Ameisensäure, Essigsäure, Propionsäure, n-Buttersäure oder n-Valeriansäure verwendet.

9. Verfahren gemäß Anspruch 1, dadurch gekennzeichnet, daß man Olefine mit 7 bis 20 C-Atomen oder Polyisobuten hydroformyliert.

## Claims

1. A process for the preparation of aldehydes or aldehydes and alcohols by hydroformylation of olefins having more than 3 carbon atoms, comprising a hydroformylation step, in which the olefin is hydroformylated at from 50 to 1000 bar and at a temperature of from 50 to 180°C by means of a rhodium catalyst dissolved in a homogenous reaction medium, and a catalyst recovery step by extraction of the rhodium catalyst, which comprises
(a) carrying out the hydroformylation in the presence of a rhodium complex which contains, as ligand, a phosphorus-free, multidentate organic nitrogen compound which is capable of forming a complex with metals from group VIII of the Periodic Table and which additionally contains at least one tertiary nitrogen radical which can be protonated by a weak acid,
(b) subjecting the product from the hydroformylation step, if necessary after removal or partial removal of the aldehydes and alcohols, to extraction with an aqueous solution of a distillable acid,
(c) subjecting the aqueous acid extract to heat treatment in the presence of an organic solvent or solvent mixture which is inert under the hydroformylation conditions, with removal of the aqueous acid by distillation, resulting in the complex being deprotonated and transferred into the organic phase, and
(d) feeding the organic phase containing the catalyst complex back into the hydroformylation step.

2. A process as claimed in claim 1, wherein use is made of distillable acids whose ammonium salts can be cleaved back into free ligand and free acid at from room temperature to 200°C.

3. A process as claimed in claim 1, wherein ligands of the formula I 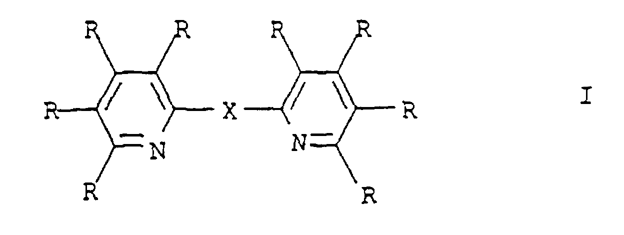 where X is a bridging unit selected from the group consisting of a covalent bond, methylene, ethylene, oxo, thio, alkylimino and arylimino, and the radicals R, independently of one another, are hydrogen, alkyl radicals having 1 to 18 carbon atoms or alkoxy radicals having 1 to 18 carbon atoms, where these radicals may be a constituent of a saturated or unsaturated ring, and where at least one of the radicals R or, where appropriate, of the substituents on a ring formed by the alkyl or alkoxy radicals is a radical of the formula II 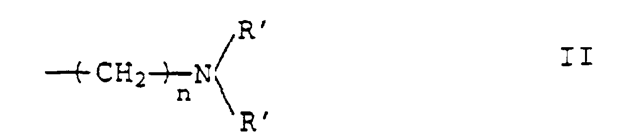 where n is a number from 0 to 20, and R' are alkyl, cycloalkyl, aralkyl or aryl radicals having up to 18 carbon atoms, where the radicals R' may be bridged with one another.

4. A process as claimed in claim 1, wherein the ligands used are polyamines of the formula III 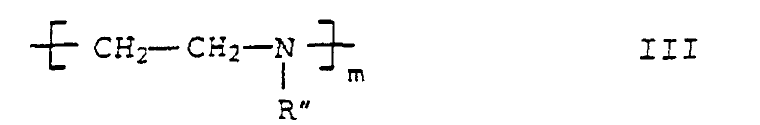 where R" is hydrogen or an alkyl, cycloalkyl, aryl or aralkyl radical, which may itself carry dialkylamino radicals, and m is a number from 2 to 35,000, with the proviso that, in the case where R" = hydrogen, at least some of the hydrogen atoms are substituted by alkylcarbonyl having 2 to 18 carbon atoms or by hydroxy (alkoxy) alkyl radicals obtained by reaction of the secondary amino group with from 1 to 10 mol of ethylene oxide or from 1 to 10 mol of propylene oxide, and with the further proviso that the polyamine contains at least three tertiary nitrogen atoms which are capable of protonation.

5. A process as claimed in claim 1, wherein the ligands used are bipyridines of the formula IV 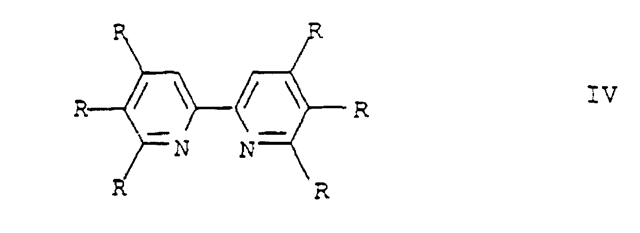 where the radicals R are as defined in claim 3 and are or carry at least one radical containing a tertiary amino group, of the formula II 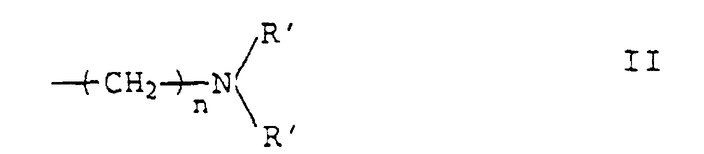 where n and R' are as defined in claim 3.

6. A process as claimed in claim 1, wherein the ligands used are compounds of the formula 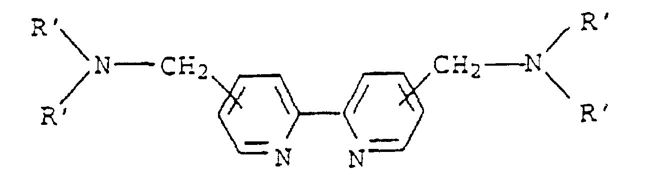 where the radical 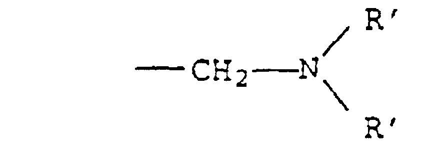 is bonded to the pyridyl rings in the 4,4'- or 5,5'-position, and in which the R', independently of one another, are alkyl, cycloalkyl, aralkyl or aryl radicals having up to 18 carbon atoms.

7. A process as claimed in claim 1, wherein the extraction is carried out using acids having pK values of from 3 to 6.

8. A process as claimed in claim 1, wherein the extraction is carried out using aqueous solutions of carbonic acid, formic acid, acetic acid, propionic acid, n-butyric acid or n-valeric acid.

9. A process as claimed in claim 1, wherein an olefin having 7 to 20 carbon atoms or polyisobutene is hydroformylated.

## Revendications

1. Procédé de préparation d'aldéhydes ou d'aldéhydes et d'alcools par hydroformylation d'oléfines ayant plus de 3 atomes de carbone, comprenant une étape d'hydroformylation dans laquelle on hydroformyle l'oléfine au moyen d'un catalyseur au rhodium dissous dans un milieu de réaction homogène, à une pression de 50 à 1 000 bars et à une température de 50°C à 180°C, et une étape de récupération du catalyseur au moyen d'une extraction du catalyseur au rhodium, caractérisé en ce que,
(a) on réalise l'hydroformylation en présence d'un complexe au rhodium, qui, en tant que ligand, comprend un composé organique azoté exempt de phosphore, polydenté, capable de former des complexes avec les métaux du Groupe VIII du Tableau Périodique, qui contient de plus au moins un résidu azoté tertiaire pouvant être protoné par un acide faible,
(b) on soumet le produit de l'étape d'hydroformylation, éventuellement après séparation ou séparation partielle des aldéhydes et des alcools, à une extraction avec une solution aqueuse d'un acide distillable,
(c) on soumet l'extrait acide aqueux en présence d'un solvant ou mélange de solvants organiques qui est inerte dans les conditions d'hydroformylation, à un traitement thermique avec élimination par distillation de l'acide aqueux, traitement par lequel le complexe est déprotoné et transformé dans la phase organique et
(d) on recycle la phase organique contenant le complexe de catalyseur dans l'étape d'hydroformylation.

2. Procédé selon la revendication 1, caractérisé en que l'on utilise des acides distillables, dont les sels d'ammonium peuvent être dissociés en ligands libres et en acide libre entre la température ambiante et 200°C.

3. Procédé selon la revendication 1, caractérisé en ce que l'on utilise des ligands de formule I 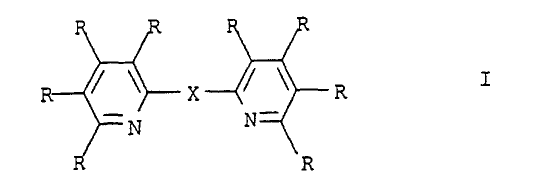 dans laquelle X représente un élément de pont, choisi dans le groupe formé par une liaison covalente, un groupe méthylène, éthylène, oxo, thio, alkylimino et arylimino et les résidus R représentent indépendamment l'un de l'autre, un atome d'hydrogène, des résidus alkyle ayant 1 à 18 atomes de C ou des résidus alcoxy ayant 1 à 18 atomes de C, ces résidus pouvant faire partie d'un cycle saturé ou insaturé et où au moins un des restes ou éventuellement un des substituants sur un cycle qui est formé par les résidus alkyle ou alcoxy, est un reste de formule II 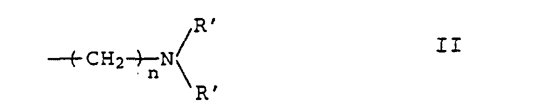 dans laquelle n représente les nombres de 0 à 20, et R' représente des résidus alkyle, cycloalkyle, aralkyle ou aryle ayant jusqu'à 18 atomes de C, où les résidus R' peuvent être pontés l'un à l'autre.

4. Procédé selon la revendication 1, caractérisé en ce que l'on utilise comme ligands, des polyamines de formule III 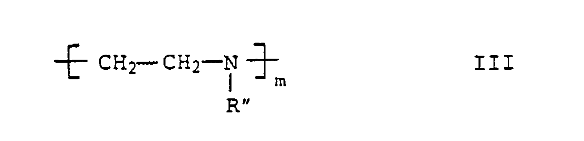 dans laquelle R'' représente un atome d'hydrogène, un groupe alkyle, cycloalkyle, aryle ou aralkyle, qui peuvent porter de leur côté des résidus dialkylamino, et m représente un nombre de 2 à 35 000, avec la condition que, dans le cas où R" représente un atome d'hydrogène, au moins une partie des atomes d'hydrogène sont substitués par un groupe alkylcarbonyle ayant 2 à 18 atomes de C ou par des résidus hydroxy(alcoxy)-alkyle, qui sont obtenus par réaction du groupe amino secondaire avec 1 à 10 moles d'oxyde d'éthylène ou 1 à 10 moles d'oxyde de propylène, et avec la condition supplémentaire que la polyamine contient au moins 3 atomes d'azote tertiaire capables de protonation.

5. Procédé selon la revendication 1, caractérisé en ce que l'on utilise comme ligands, des bipyridines de formule IV 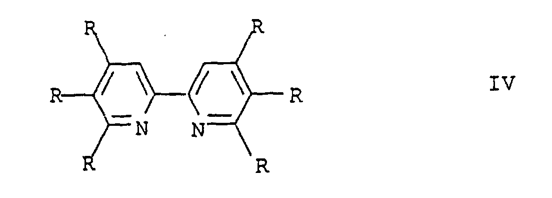 dans laquelle, les résidus prennent les significations indiquées dans la revendication 3 et qui portent au moins un résidu contenant un groupe amino tertiaire de formule II 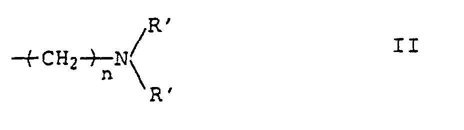 dans laquelle n et R' prennent les significations indiquées dans la revendication 3.

6. Procédé selon la revendication 1, caractérisé en ce que l'on utilise comme ligands, des composés de formules 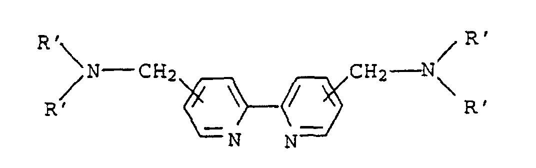 dans laquelle le résidu 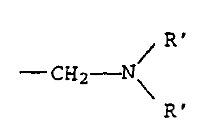 est lié au cycle pyridyle dans la position 4,4' ou 5,5' et dans laquelle les R' représentent indépendamment l'un de l'autre des résidus alkyle, cycloalkyle, aralkyle ou aryle ayant jusqu'à 18 atomes de C.

7. Procédé selon la revendication 1, caractérisé en ce que l'on utilise pour l'extraction, des acides ayant des valeurs de pK de 3 à 6.

8. Procédé selon la revendication 1, caractérisé en ce que l'on utilise pour l'extraction, des solutions aqueuses de l'acide carbonique, de l'acide formique, de l'acide acétique, de l'acide propionique, de l'acide n-butyrique ou de l'acide n-valérique.

9. Procédé selon la revendication 1, caractérisé en ce que l'on hydroformyle des oléfines ayant 7 à 20 atomes de C ou du polyisobutène.
